# EUROPÄISCHE PATENTSCHRIFT

(11) **EP 0 522 476 B1**
(45) Veröffentlichungstag und Bekanntmachung des Hinweises auf die Patenterteilung: **27.12.1995**
(21) Anmeldenummer: 92111383.3
(22) Anmeldetag: 04.07.1992
(51) Int. Cl.: C07C 51/487, C07C 67/60, C07C 209/86

(54) **Verfahren zur Abtrennung aliphatischer geradkettiger Verbindungen mit endständigen funktionellen Gruppen**
Method for separating linear aliphatic compounds terminated by fonctional groups
Procédé de séparation de composés linéaires aliphatiques ayant des groupes fonctionnels en position terminale

(30) Priorität: 12.07.1991 DE 4123084
(43) Veröffentlichungstag der Anmeldung: 13.01.1993
(73) Patentinhaber: HOECHST AKTIENGESELLSCHAFT, 65926 Frankfurt am Main (DE)
(72) Erfinder: Deckers, Gregor, Dr., W-4232 Xanten (DE); Frohning, Dieter, Dr., W-4230 Wesel (DE)

(56) Entgegenhaltungen:
- EP-A- 0 415 697
- CHEMICAL ABSTRACTS, vol. 80, no. 9, 4. März 1974, Columbus, Ohio, US; abstract no. 47417h, E. MANNIK ET AL 'Use of inclusion compounds with urea for the separation of dimethyl esters of normal dicarboxylic acids and alpha-methyl isomers' Seite 315 ;
- INDUSTRIAL AND ENGINEERING CHEMISTRY Bd. 42, Nr. 7, Juli 1950, COLUMBUS US Seiten 1300 - 1306 W. J. ZIMMERSCHEID ET AL. 'Crystalline Adducts of Urea with Linear Aliphatic Compounds'

## Beschreibung

Die vorliegende Erfindung betrifft ein Verfahren zur Abtrennung aliphatischer geradkettiger Verbindungen mit endständigen funktionellen Gruppen aus Gemischen, die Isomere enthalten. Derartige Gemische lassen sich nicht immer mit den üblichen Methoden in ihre Einzelkomponenten auftrennen. Aufgrund der sehr nahe beieinanderliegenden Siedepunkte isomerer Verbindungen erweist sich eine Destillation häufig als nicht geeignet, aliphatische geradkettige Verbindungen mit endständigen funktionellen Gruppen aus Isomeren enthaltenden Gemischen abzutrennen. Wegen der recht geringen Siedepunktunterschiede aliphatischer Isomerer sind Destillationskolonnen mit großer Bödenzahl erforderlich. Um die Trennschärfe der Kolonnen sinnvoll auszunutzen, ist die Destillation mit einem hohem Rücklaufverhältnis durchzuführen, was zu einer langen Aufenthaltszeit der abzutrennenden Einzelkomponenten in den Kolonnen und somit zu einer erheblichen thermischen Belastung des Destillationsgutes führt. Eine hohe thermische Belastung hat in der Regel eine Minderung der Ausbeute zur Folge und erweist sich insbesondere bei empfindlichen Verbindungen wegen Entstehung von Verfärbungen, die destillativ nicht mehr zu entfernen sind, und wegen Bildung unerwünschter Nebenprodukte als nachteilig. Die Destillation läßt sich als Trennmethode allerdings nur auf destillierbare Stoffe anwenden. Substanzen, die infolge einer Destillation einen Abbau oder eine thermische Zersetzung erleiden, eignen sich hingegen nicht für eine destillative Abtrennung.

Auch die fraktionierte Kristallisation, eine ebenfalls übliche Trennmethode, läßt sich nur begrenzt anwenden. Zum einen ist dieses Verfahren auf kristallisierbare Stoffe beschränkt und zum anderen erfordert die fraktionierte Kristallisation insbesondere wegen der zahlreichen einzelnen Kristallisationsschritte einen hohen Arbeitseinsatz, der mit einem großen apparativen Aufwand verbunden ist.

Die Isolierung und Reinigung von methylverzweigten gesättigten Fettsäuren mit 14 bis 24 Kohlenstoffatomen mittels einer besonderen Variante der fraktionierten Kristallisation wird in der DE 38 07 401 A1 beschrieben. Man versetzt die geschmolzene Fettsäuremischung mit einer wäßrigen Lösung eines Netzmittels und läßt unter Rühren kristallisieren. Dieser Vorgang wird als Umnetztrennung oder auch als fraktionierte Kristallisation in Anwesenheit von Netzmitteln bezeichnet. Die dabei anfallende Dispersion wird zentrifugiert, wobei eine Auftrennung in eine weitgehend netzmittelfreie methylverzweigte Fettsäure und eine schwerere aus Netzmittellösung und darin dispergierte Kristalle geradkettiger gesättigter Fettsäuren bestehende Phase erfolgt.

In begrenztem Umfange finden auch spezielle Methoden zur Trennung und Anreicherung aliphatischer Verbindungen Anwendung. So beschreiben Zimmerschied, Dinerstein, Weitkamp und Marschner in Ind. Eng. Chem. 1950, 42(7), Seiten 1300 bis 1306 kristalline Addukte von Harnstoff mit linearen aliphatischen Verbindungen. Die Bildung von Harnstoffeinschlußverbindungen ist von unterschiedlichen Faktoren abhängig. Hierzu zählen die Kettenlänge und Linearität der aliphatischen Verbindung. Aber auch die Art und Größe von Substituenten, so wie deren Anzahl und Stellung üben einen Einfluß auf die Bildung von Harnstoffeinschlußverbindungen aus. Bei ausreichender Kettenlänge führen auch gering verzweigte Alkane zu Harnstoffaddukten. So führt beispielsweise 2-Methyloctadecan zu einer Harnstoffeinschlußverbindung, deren Stabilität mit der von n-Hexadecan vergleichbar ist. Ein ähnliches Verhalten zeigen auch die Ester von Fettsäuren. So bilden auch Ester von methylverzweigten Carbonsäuren Harnstoffeinschlußverbindungen (Vergl. Seite 1302 linke Spalte bis Seite 1303 linke Spalte, 2. Absatz).

E. Mannik, O. Ikonopistseva, A. Fomina und A. Mannik beschreiben in Nachrichten der Akademie der Wissenschaften der Estnischen SSR, Band 22, Chemie-Geologie, 1973, Nr. 4, S. 283 - 287 den Einsatz von Harnstoffeinschlußkomplexen zur Abtrennung von normalen Dicarbonsäuredimethylestern der Formel CH₃OOC-(CH₂)ₙ-COOCH₃ mit n = 2 - 5, 7 und 8 von den entsprechenden α-Methyl-Isomeren.

Die GB-PS 1 240 513 beschreibt die Gewinnung einer aus Estern der Linolsäure und γ-Linolensäure bestehenden therapeutisch verwendbaren Mischung. Durch Zusatz von Harnstoff zu einer Lösung von Estern der Palmitin-, Stearin-, Öl-, Linol- und γ-Linolensäure in Methanol scheidet man als Harnstoffeinschlußverbindungen ein Gemisch überwiegend von Estern der Palmitin-, Stearin- und Ölsäure ab. Die gewünschte therapeutisch verwendbare Mischung von Estern der Linol- und γ-Linolensäure erhält man durch anschließende Extraktion der verbleibenden Lösung mit einem geeigneten organischen Lösungsmittel. Diese Arbeitsweise beschränkt sich auf die Auftrennung von Estern unverzweigter gesättigter und ungesättigter Carbonsäuren.

Der vorliegenden Erfindung liegt die Aufgabe zugrunde, ein Verfahren, das die vorstehend geschilderten Nachteile vermeidet und sich zugleich mit einem relativ geringfügigen Aufwand durchführen läßt, bereitzustellen. Das Verfahren soll darüberhinaus eine gute Abtrennung der gewünschten Verbindungen gewährleisten.

Diese Aufgabe wird gelöst durch ein Verfahren zur Abtrennung aliphatischer, geradkettiger Verbindungen mit 10 bis 19 Kohlenstoffatomen in der unverzweigten Kette und endständigen funktionellen Gruppen aus in α-Stellung verzweigte Isomere enthaltenden Gemischen, dadurch gekennzeichnet, daß man das die aliphatische Verbindung enthaltende Gemisch mit einem Lösungsmittel und Harnstoff versetzt, gegebenenfalls erwärmt, durch anschließendes Abkühlen eine Harnstoffeinschlußverbindung (Clathrat) auskristallisieren läßt und die aliphatische Verbindung aus der kristallisierten Harnstoffeinschlußverbindung freisetzt.

Im Gegensatz zu reinem Harnstoff, der tetragonale Prismen bildet, kristallisieren Harnstoffclathrate in einem hexagonalen Gitter, das lange zusammenhängende Kanäle enthält. In diese Kanäle werden die Fremdstoffmoleküle, auch Gastmoleküle bezeichnet, eingelagert. Die Harnstoffmoleküle bauen sechsseitige Prismen mit einem Querschnitt ähnlich einer Bienenwabe auf.

Aufgrund stark ausgeprägter van der Waalsscher Kräfte zwischen Harnstoff und Gastmolekül stabilisieren die Gastmoleküle das hexagonale Gitter des Clathrats. Die Fremdstoffmoleküle sind nicht immer einer besonderen räumlichen Anordnung folgend eingebaut. Langkettige Kohlenwasserstoffe und deren Derivate werden üblicherweise mit der Längsachse in die Achse der sechsseitigen Prismen eingelagert.

Das hexagonale Gitter ist für sich betrachtet nicht beständig und bricht zusammen, sobald die eingebauten Moleküle durch Verdampfen oder Extrahieren entfernt werden. Auf diese Weise läßt sich die als Gastmolekül eingebaute aliphatische Verbindung aus der kristallisierten Harnstoffeinschlußverbindung wieder freisetzen.

Die als Clathrat abzutrennende aliphatische Verbindung soll eine unverzweigte Kette ausreichender Länge aufweisen. Demzufolge sollte die Kette wenigstens 8 Kohlenstoffatome umfassen.

Geeignet sind aliphatische geradkettige Verbindungen mit 10 bis 19 Kohlenstoffatomen in der unverzweigten Kette.

Das erfindungsgemäße Verfahren läßt sich zur Abtrennung von aliphatischen, geradkettigen Verbindungen anwenden, die als endständige funktionelle Gruppe an einem oder beiden Enden der Kohlenstoffkette einen Alkohol-, Amin-, Ether-, Carbonsäure- oder Carbonsäureesterrest, insbesondere einen Amin-, Carbonsäure- oder Carbonsäureesterrest, besonders bevorzugt einen Carbonsäurerest, aufweist.

Als Alkohole sind n-Undecanol, n-Hexadecanol, n-Octadecanol und n-Nonadecanol, insbesondere n-Undecanol und n-Nonadecanol, zu nennen.

Beispiele für Amine ist 1,12-Diaminododecan und für Ether n-Alkyl-tert.-butylether, worin für n-Alkyl eine Gruppe mit 8 bis 24 C-Atomen steht.

Für Carbonsäuren stehen Undecan-, Dodecan-, Tridecan-, Pentadecan-, Hexadecan-, Octadecan- und Nonadecansäure, insbesondere Undecan-, Tridecan-, Pentadecan- und Nonadecansäure.

Als Carbonsäureester sind die Methyl-, Ethyl- und Propylester gesättigter, unverzweigter Carbonsäuren, beispielsweise die der Decan-, Undecan-, Dodecan-, Tridecan-, Pentadecan-, Hexadecan-, Octadecan- und Nonadecansäure, insbesondere Undecan-, Tridecan-, Tetradecan- und Nonadecansäure zu erwähnen.

Aus den vorstehend genannten aliphatischen, geradkettigen Verbindungen sind Undecanol, Nonadecanol, Pentadecansäure, Pentadecansäuremethylester, insbesondere Pentadecansäure oder Pentadecansäuremethylester, als brauchbar hervorzuheben.

Bei den abzutrennenden Isomeren handelt es sich neben üblichen Stellungsisomeren um solche, die in α-Stellung, also in unmittelbarer Nachbarschaft zur endständigen funktionellen Gruppe der aliphatischen geradkettigen Verbindung einer Methylgruppe, besitzen.

Bedingt durch die Art der Herstellung enthalten bestimmte Reaktionsprodukte in erheblichem Umfang α-methylverzweigte Isomere. Dies trifft in besonderem Maß auf Stoffe zu, die mittels Hydroformylierung aus endständigen Olefinen hergestellt und gegebenenfalls anschließend weiterverarbeitet werden. Die aus der Hydroformylierung hervorgehenden Aldehyde bestehen in der Regel aus einem Gemisch geradkettiger unverzweigter und in α-Stellung methylverzweigter Aldehyde. Derartige Aldehydgemische lassen sich zu entsprechenden Alkohol-, Carbonsäure- und Carbonsäureestergemischen umsetzen.

Man versetzt das die abzutrennenden Verbindungen enthaltende Gemisch mit einem Lösungsmittel und Harnstoff. Als Lösungsmittel läßt sich ein Alkohol, ein Keton, ein Ether, ein Kohlenwasserstoff und/oder Wasser verwenden. Geeignete Alkohole sind aliphatische Alkohole, insbesondere aliphatische Alkohole mit 1 bis 4 Kohlenstoffatomen, bevorzugt Methanol und/oder Ethanol gegebenenfalls im Gemisch mit Wasser. Besonders brauchbar ist Methanol.

In zahlreichen Fällen kann der Gebrauch von Wasser als Lösungsmittel erforderlichenfalls zusammen mit einem oder mehreren der vorstehend genannten Solventien hilfreich sein.

Die Wahl des Lösungsmittels respektive des Lösungsmittelgemisches ist abhängig von der Art des Abtrennungsproblemes. Daher ist ein für das jeweils durchzuführende Trennungsverfahren geeignetes Lösungsmittel oder Lösungsmittelgemisch auszusuchen. Das Lösungsmittel oder das Lösungsmittelgemisch soll mit Harnstoff keine Clathrate oder Addukte bilden und sich gegenüber den in Lösung befindlichen Stoffen inert verhalten.

In den meisten Fällen hat sich die Verwendung von Methanol, Ethanol und/oder Wasser bewährt.

Lösungsmittel und Harnstoff können dem zu trennenden Gemisch getrennt und gleichzeitig oder aufeinander folgend zugesetzt werden. Üblicherweise setzt man zunächst das Lösungsmittel und anschließend den Harnstoff unter Rühren zu. Es ist auch möglich, Harnstoff in Form einer Lösung zu verwenden.

Für die Durchführung des erfindungsgemäßen Verfahrens ist die Menge Harnstoff, bezogen auf die als Clathrat abzutrennende Verbindung, von Bedeutung. Eine sinkende Menge Harnstoff zieht zwar eine Steigerung der Selektivität des Trennverfahrens aber zugleich auch eine Herabsetzung der Ausbeute nach sich. Eine Erhöhung der eingesetzten Menge Harnstoff führt einerseits zu einer Anhebung der Ausbeute, andererseits jedoch zu einer Verringerung der Selektivität des Trennverfahrens.

Bei Festlegung der einzusetzenden Harnstoffmenge erweist es sich als nützlich, neben der Molmenge der abzutrennenden Verbindung auch die Kettenlänge des betreffenden Moleküls zu berücksichtigen. Als Richtwert empfiehlt es sich, so viel Harnstoff zu verwenden, daß je Å (10⁻¹⁰ m) der Molekülkette, die als Gastmolekül in das Harnstoffgerüst eingefügt wird, 0,3 bis 1,0, insbesondere 0,4 bis 0,9, bevorzugt 0,5 bis 0,75 Einheiten Harnstoff zur Verfügung stehen. So erfordert ein Mol einer aliphatischen Verbindung mit 12 Å Kettenlänge die 12-fache Menge der vorstehend genannten Richtwerte gerechnet als Mol Harnstoff. Hingegen benötigen 0,5 Mol einer Verbindung mit einer Kettenlänge von beispielsweise 16 Å nur die 8-fache Menge des üblichen Richtwertes gerechnet als Mol Harnstoff.

Die Kettenlänge von Alkanen mit 7, 10, 12 bzw. 16 Kohlenstoffatomen beträgt etwa 9, 13,5, 15,5 bzw. 21 Å, die Kettenlänge von Carbonsäuren mit 7, 10, 12, 16 bzw. 18 Kohlenstoffatomen ist mit etwa 10, 14, 16,5, 21,5 bzw. 24 Å zu veranschlagen. Die Kettenlängen von Carbonsäuremethylestern und Alkoholen liegen in vergleichbaren Größenordnungen. Anhand dieser Werte kann eine relativ genaue Abschätzung des Harnstoffbedarfes vorgenommen werden (vergl. Schenck, Ann. 565, 1949, S. 204 bis 240).

Das erfindungsgemäße Verfahren läßt sich sowohl absatzweise als auch kontinuierlich durchführen. Besonders einfach gestaltet sich eine absatzweise Durchführung.

Üblicherweise setzt man den Harnstoff oder die den Harnstoff enthaltende Lösung dem aufzutrennenden Gemisch in der gewünschten Menge zu, erhöht, gegebenenfalls unter Rühren, die Temperatur, bis man eine klare Lösung erhält und kühlt diese anschließend ab.

Es hat sich im allgemeinen bewährt, nach Zugabe des Harnstoffes die Mischung auf eine Temperatur von 25 bis 100, insbesondere 30 bis 90, bevorzugt 35 bis 85 °C zu erwärmen. Rühren unterstützt das Auflösen des Harnstoffes.

Anschließend laßt man die Harnstoffeinschlußverbindungen enthaltende Lösung langsam auf beispielsweise -25 bis +50, insbesondere -10 bis +40, bevorzugt 0 bis +30°C abkühlen. Die kristallisierten Harnstoffclathrate werden beispielsweise durch Dekantieren und/oder Filtrieren abgetrennt und gegebenenfalls mit kaltem Lösungsmittel oder Lösungsmittelgemisch gewaschen.

Die als Clathrat abgetrennte aliphatische, geradkettige Verbindung kann durch Lösen der Harnstoffeinschlußverbindung in Wasser freigesetzt und gegebenenfalls nach Ansäuern durch Extraktion mittels eines organischen Lösungsmittels aus der wäßrigen Lösung entfernt werden. Als Lösungsmittel eignen sich in Wasser unlösliche organische Solventien, wie Cyclohexan, Essigsäureethylester, Di-n-butylether, insbesondere Essigsäureethylester.

Falls erforderlich, kann das vorstehend beschriebene Verfahren mehrfach wiederholt werden.

Die nachfolgend beschriebenen Beispiele belegen die Erfindung, ohne sie hierauf zu beschränken.

### Experimenteller Teil

### Beispiel 1

3,85 g eines Gemisches, das 57,65 Masse-% n-Pentadecansäure und 18,12 Masse-% 2-Methyltetradecansäure enthält, werden mit 8,2 g Harnstoff und 25 ml eines aus 8 Volumteilen Isopropanol und 1 Volumteil Wasser bestehenden Lösungsmittelgemisches versetzt und unter Rühren erhitzt, bis eine klare Lösung erhalten wird.

Man kühlt auf 20°C ab und läßt den Ansatz 24 Stunden bei dieser Temperatur stehen. Anschließend filtriert man die ausgefallenen Clathratkristalle mit Hilfe einer Filternutsche ab und wäscht den Filterrückstand dreimal mit jeweils 10 ml eines auf 5°C abgekühlten aus 8 Volumteilen Isopropanol und 1 Volumteil Wasser bestehenden Lösungsmittelgemisches.

Die Clathratkristalle werden in Wasser gelöst. Durch Zusatz von Salzsäure wird ein pH-Wert von 3 bis 4 eingestellt. Die angesäuerte wäßrige Lösung wird dreimal mit jeweils 20 ml Cyclohexan versetzt und extrahiert. Die abgetrennten Cyclohexanphasen werden vereinigt, zweimal mit Wasser gewaschen und anschließend über Na₂SO₄ getrocknet. Nach Entfernen des Cyclohexans durch Abdampfen erhalt man ein Gemisch, das 66,0 Masse-% n-Pentadecansäure, aber lediglich 2,15 Masse-% 2-Methyltetradecansäure aufweist.

60,9 Masse-% der eingesetzten n-Pentadecansäure werden zurückgewonnen.

Die analytische Bestimmung der Carbonsäuren erfolgt in allen Beispielen nach Überführung in die entsprechenden Methylester mittels gaschromatographischer Analyse. Stearinsäure dient dabei als innerer Standard.

### Beispiel 2

24,2 g des in Beispiel 1 eingesetzten Gemisches werden mit 72 g Harnstoff und 180 ml Methanol versetzt, auf 60°C erhitzt und gerührt, bis eine klare Lösung erhalten wird. Man kühlt auf Raumtemperatur ab und läßt den Ansatz 24 Stunden bei dieser Temperatur stehen. Anschließend filtriert man die ausgefallenen Clathratkristalle mit Hilfe einer Filternutsche ab.

Für analytische Zwecke werden ca. 5 Masse-% der auf diese Weise gewonnenen Clathratkristalle entnommen und, wie nachfolgend angegeben, aufgearbeitet. Die verbleibende Menge wird, wie in Beispiel 3 angegeben, weiterverarbeitet.

Die Clathratkristalle werden in Wasser gelöst. Durch Zusatz von Salzsäure wird ein pH-Wert von 3 bis 4 eingestellt. Die angesäuerte wäßrige Lösung wird dreimal mit jeweils 100 ml Essigsäureethylester versetzt und extrahiert. Die abgetrennten Essigsäureethylesterphasen werden vereinigt, zweimal mit Wasser gewaschen und anschließend über Na₂SO₄ getrocknet. Nach Entfernen des Essigsäureethylesters durch Abdampfen erhält man ein Gemisch, das 66,1 Masse-% n-Pentadecansäure, aber lediglich 1,0 Masse-% 2-Methyltetradecansäure aufweist.

### Beispiel 3

Die aus Beispiel 2 entstammenden Clathratkristalle, aus denen sich ein Gemisch, das 1,0 Masse-% 2-Methyltetradecansäure und 66,1 Masse-% n-Pentadecansäure enthält, gewinnen läßt, werden mit 21 g Harnstoff und 180 ml Methanol versetzt, auf 60°C erhitzt und gerührt, bis eine klare Lösung erhalten wird. Man kühlt auf Raumtemperatur ab und läßt den Ansatz 24 Stunden bei dieser Temperatur stehen. Anschließend filtriert man die ausgefallenen Clathratkristalle mit Hilfe einer Filternutsche ab.

Die Clathratkristalle werden in Wasser gelöst, und es wird - wie in Beispiel 2 angegeben - weitergearbeitet. Nach Zusatz von Salzsäure, Extraktion mittels Essigsäureethylester, Trocknung der vereinigten organischen Phasen und Abdampfen des Essigsäureethylesters erhält man ein Gemisch, das 76,1 Masse-% n-Pentadecansäure, aber lediglich 0,22 Masse-% 2-Methyltetradecansäure aufweist.

Die Ausbeute an n-Pentadecansäure beträgt, gaschromatographisch ermittelt, 74,7 Masse-% und, gravimetrisch bestimmt, 78,9 Masse-%, jeweils bezogen auf die in Beispiel 2 eingesetzte Menge n-Pentadecansäure. Die Ausbeute an n-Pentadecansäure beträgt, jeweils auf die in Beispiel 3 eingesetzte Menge n-Pentadecansäure bezogen, gaschromatographisch ermittelt, 80,1 Masse-% und, gravimetrisch bestimmt, 84,6 Masse-%.

### Beispiel 4

90,3 g eines Gemisches, das 52,77 Masse-% n-Pentadecansäuremethylester und 17,71 Masse-% 2-Methyltetradecansäuremethylester enthält, werden in drei aufeinanderfolgenden Stufen aufgearbeitet.

Das Methylestergemisch sowie die aus den Stufen 1 und 2 resultierenden Clathratkristalle werden mit der aus der nachfolgenden Tabelle jeweils zu entnehmenden Menge Harnstoff und Methanol versetzt, auf Rückflußtemperatur erhitzt und gerührt, bis eine klare Lösung erhalten wird.

Man kühlt auf Raumtemperatur ab und läßt den Ansatz 24 Stunden bei dieser Temperatur stehen, kühlt danach für weitere 6 Stunden auf +5°C und filtriert die ausgefallenen Clathratkristalle mit Hilfe einer Filternutsche ab.

Die in Stufe 3 anfallenden Clathratkristalle werden in Wasser gelöst. Durch Zusatz von Salzsäure wird ein pH-Wert von 3 bis 4 eingestellt. Die angesäuerte wäßrige Lösung wird dreimal mit jeweils 100 ml Essigsäureethylester versetzt und extrahiert. Die abgetrennten Essigsäureethylesterphasen werden vereinigt, zweimal mit Wasser gewaschen und anschließend über Na₂SO₄ getrocknet. Nach Entfernen des Essigsäureethylesters durch Abdampfen erhält man ein in der nachfolgenden Tabelle ausgewiesenes Gemisch (bestimmt durch gaschromatographische Analyse). Die Ausbeuteangaben wurden gravimetrisch ermittelt.

Für analytische Zwecke werden jeweils ca. 5 Masse-% der in den einzelnen Stufen anfallenden Clathratkristalle entnommen und analog zu den in Stufe 3 anfallenden Clathratkristallen aufgearbeitet.

Die verbleibende Menge der aus den Stufen 1 und 2 anfallenden Clathrate wird in die Stufen 2 und 3 eingesetzt und, wie in der vorangegangenen Vorschrift vorgesehen, weiterverarbeitet. Bei den Angaben bezüglich der Ausbeute ist die für analytische Zwecke abgezweigte Menge berücksichtigt.

**Tabelle**

| Stufe | 1 | 2 | 3 |
|---|---|---|---|
| Harnstoff (g) | 271 | 50 | 40 |
| Methanol (ml) | 900 | 750 | 600 |
| Einsatzstoff (g) | 90,3 | (Produkt aus Stufe 1) | (Produkt aus Stufe 2) |
| 2-Methyltetradecansäuremethylester (Masse-%) | 6,62 | 1,75 | 0,07 |
| n-Pentadecansäuremethylester (Masse-%) | 66,6 | 68,5 | 67,2 |
| Ausbeute (Masse-%) | 91,6 | 86,3* | 75,8* |

| | | | |
|---|---|---|---|
| * Ausbeute bezogen auf die in Stufe 1 eingesetzte Menge n-Pentadecansäuremethylester | | | |

## Patentansprüche

1. Verfahren zur Abtrennung aliphatischer geradkettiger Verbindungen mit 10 bis 19 Kohlenstoffatomen in der unverzweigten Kette und endständigen funktionellen Gruppen aus in α-Stellung methylverzweigte Isomere enthaltenden Gemischen, dadurch gekennzeichnet, daß man das die aliphatische Verbindung enthaltende Gemisch mit einem Lösungsmittel und Harnstoff versetzt, gegebenenfalls erwärmt, durch anschließendes Abkühlen eine Harnstoffeinschlußverbindung (Clathrat) auskristallisieren läßt und die aliphatische Verbindung aus der kristallisierten Harnstoffeinschlußverbindung freisetzt.

2. Verfahren nach Anspruch 1, dadurch gekennzeichnet, daß die aliphatische geradkettige Verbindung als endständige funktionelle Gruppe einen Alkohol-, Amin-, Ether-, Carbonsäure- oder Carbonsäureesterrest enthält.

3. Verfahren nach einem oder mehreren der Ansprüche 1 bis 2, dadurch gekennzeichnet, daß die aliphatische geradkettige Verbindung Undecanol, Nonadecanol, 1,8-Diaminooctan, Pentadecansäure oder Pentadecansäuremethylester ist.

4. Verfahren nach einem oder mehreren der Ansprüche 1 bis 3, dadurch gekennzeichnet, daß als Lösungsmittel ein Alkohol, ein Keton, Ether, Kohlenwasserstoff und/oder Wasser, verwendet wird.

5. Verfahren nach einem oder mehreren der Ansprüche 1 bis 4, dadurch gekennzeichnet, daß man je Mol abzutrennender aliphatischer geradkettiger Verbindung und je Å der im Harnstoff eingeschlossenen Molekülkette der abzutrennenden aliphatischen geradkettigen Verbindung 0,3 bis 1,0 Mol Harnstoff einsetzt.

6. Verfahren nach einem oder mehreren der Ansprüche 1 bis 5, dadurch gekennzeichnet, daß man auf eine Temperatur von 25 bis 100°C erwärmt.

7. Verfahren nach einem oder mehreren der Ansprüche 1 bis 6, dadurch gekennzeichnet, daß man die Harnstoffeinschlußverbindungen enthaltende Lösung langsam auf -25 bis +50°C abkühlt, die kristallisierten Harnstoffeinschlußverbindungen abtrennt und gegebenenfalls mit kaltem Lösungsmittel wäscht.

8. Verfahren nach einem oder mehreren der Ansprüche 1 bis 7, dadurch gekennzeichnet, daß man die aliphatische geradkettige Verbindung durch Lösen der Harnstoffeinschlußverbindung in Wasser freisetzt und durch Extraktion mit einem organischen Lösungsmittel aus der wäßrigen Lösung abtrennt.

## Claims

1. A process for separating aliphatic straight-chain compounds having 10 to 19 carbon atoms in the unbranched chain and terminal functional groups of mixtures containing methyl-branched isomers in the α-position, characterised in that the mixture containing the aliphatic compound is mixed with a solvent and urea, optionally heated, a urea inclusion compound (clathrate) is crystallised out by subsequent cooling, and the aliphatic compound is released from the crystallised urea inclusion compound.

2. A process according to claim 1, characterised in that the aliphatic straight-chain compound contains an alcohol, amine, ether, carboxylic acid or carboxylic acid ester radical as the terminal functional group.

3. A process according to one or both of claims 1 and 2, characterised in that the aliphatic straight-chain compound is undecanol, nonadecanol, 1,8-diaminooctane, pentadecanoic acid or pentadecanoic acid methyl ester.

4. A process according to one or more of claims 1 to 3, characterised in that an alcohol, a ketone, ether, hydrocarbon and/or water is used as the solvent.

5. A process according to one or more of claims 1 to 4, characterised in that 0.3 to 1.0 mol of urea is used per mole of aliphatic straight-chain compound to be separated and per Å of the molecule chain enclosed in the urea of the aliphatic straight-chain compound to be separated.

6. A process according to one or more of claims 1 to 5, characterised in that the mixture is heated to a temperature of 25 to 100°C.

7. A process according to one or more of claims 1 to 6, characterised in that the solution containing urea inclusion compounds is slowly cooled to -25 to + 50°C, the crystallised urea inclusion compounds are separated and, optionally, washed with cold solvent.

8. A process according to one or more of claims 1 to 7, characterised in that the aliphatic straight-chain compound is released by dissolving the urea inclusion compound in water and separated from the aqueous solution by means of extraction with an organic solvent.

## Revendications

1. Procédé pour la séparation de composés aliphatiques à chaîne droite contenant 10 à 19 atomes de carbone dans une chaîne non ramifiée et portant des groupes fonctionnels terminaux à partir de mélanges contenant des isomères à ramification méthyle en position α, caractérisé en ce que l'on ajoute un solvant et de l'urée au mélange contenant le composé aliphatique, le cas échéant on chauffe, on fait cristalliser par refroidissement un composé d'inclusion de l'urée (clathrate) et on libère le composé aliphatique du composé d'inclusion de l'urée qui a cristallisé.

2. Procédé selon la revendication 1, caractérisé en ce que le composé aliphatique à chaîne droite contient en tant que groupe fonctionnel terminal un radical d'alcool, d'amine, d'éther, d'acide carboxylique ou d'ester d'acide carboxylique.

3. Procédé selon une ou plusieurs des revendications 1 et 2, caractérisé en ce que le composé aliphatique à chaîne droite est l'undécanol, le nonadécanol, le 1,8-diaminooctane, l'acide pentadécanoïque ou le pentadécanoate de méthyle.

4. Procédé selon une ou plusieurs des revendications 1 à 3, caractérisé en ce que le solvant utilisé est un alcool, une cétone, un éther, un hydrocarbure et/ou l'eau.

5. Procédé selon une ou plusieurs des revendications 1 à 4, caractérisé en ce que, pour une mole du composé aliphatique à chaîne droite à séparer et pour 1 Å de la chaîne moléculaire, incluse dans l'urée, du composé aliphatique à chaîne droite à séparer, on met en oeuvre 0,3 à 1,0 mol d'urée.

6. Procédé selon une ou plusieurs des revendications 1 à 5, caractérisé en ce que l'on chauffe à une température de 25 à 100°C.

7. Procédé selon une ou plusieurs des revendications 1 à 6, caractérisé en ce que l'on refroidit lentement la solution contenant les composés d'inclusion de l'urée jusqu'à un niveau de -25 à +50°C, on sépare les composés d'inclusion de l'urée qui ont cristallisé et, le cas échéant, on les lave avec du solvant froid.

8. Procédé selon une ou plusieurs des revendications 1 à 7, caractérisé en ce qu'on libère le composé aliphatique à chaîne droite par dissolution du composé d'inclusion de l'urée dans l'eau et on le sépare de la solution aqueuse par extraction à l'aide d'un solvant organique.
